# DEMANDE DE BREVET EUROPEEN

(11) **EP 2 314 312 A2**
(43) Date de publication de la demande: **27.04.2011**
(21) Numéro de dépôt: 10183961.1
(22) Date de dépôt: 09.04.2003
(51) Int. Cl.: A61K 39/00, C07K 14/57, A61K 38/19, A61P 37/00

(54) **Peptides et leur application en thérapeutique**

(30) Priorité: 10.04.2002 FR 0204464
(62) Demande divisionnaire de: 03740584.2
(71) Demandeur: Vaxconsulting, 75008 Paris (FR)
(72) Inventeur: Zagury, Jean-François, 75003, PARIS (FR)
(74) Mandataire: Cabinet Plasseraud

(57) **Abrégé**

Peptide de taille comprise entre 5 et 40 acides aminés, provenant d'une cytokine, dans lequel au moins un de ses acides aminés comporte au moins un de ses atomes espacé d'une distance d de moins de 5 angströms d'un atome du récepteur correspondant à ladite cytokine, l'espacement d étant évalué sur des données structurales, dérivés, composé immunogènes les comprenant, utilisation d'un peptide ou dérivé d'un peptide ou composé immunogène pour la préparation d'un médicament curatif ou préventif destiné au traitement ou à la prévention des affections liées à un excès ou à la présence de cytokines ou au traitement d'une maladie auto-immune et compositions pharmaceutiques qui renferment au moins un peptide ou dérivé d'un peptide ou composé immunogène ci-dessus à titre de principe actif.

## Description

La présente invention concerne de nouveaux peptides et leur application en thérapeutique.

L'immunisation active anti-cytokine est une stratégie d'immunothérapie active développée depuis 1990 par Zagury et al. qui repose notamment sur la demande de brevet WO 92/22577.

Cette idée a été reprise par plusieurs équipes scientifiques qui ont publié dans des revues scientifiques internationales des immunisations actives contre la protéine entière d'IFNα multimérisée par traitement au glutaraldéhyde (Gringeri et al, JAIDS 1999;20:358-70), une protéine TNFα chimérique consistant au couplage de la protéine native du TNFα avec un épitope T de l'ovalbumine (Dalum et al, Nature Biotechnology, 1999;17:666-69), contre de l'IL9 entière couplée avec du KLH (Richard et al, PNAS, 2000;97:767-72) ou encore de l'IL5 entière chimérique avec un épitope T de toxine tétanique (Hertz et al, J. Immunol, 2001;167:3792-99).

Ces approches ont confirmé la faisabilité d'immunisations autologues anti-cytokines, mais ces quelques succès cachent les essais infructueux décrits par certains auteurs : certaines cytokines ne permettent pas d'obtenir d'anticorps suffisamment protecteurs et d'effet clinique, et la même cytokine préparée sous une forme efficace d'une façon ne le sera pas sous une autre (Richard et al, PNAS, 2000;97:767-72).

En essayant d'expliquer ce phénomène, la demanderesse a observé que jusqu'à présent tous les auteurs ont utilisé des cytokines entières (éventuellement légèrement modifiées), ce qui entraîne des difficultés notamment aux niveaux suivants :
- dilution du pouvoir immunogène des déterminants antigéniques d'intérêt (pour les réponses B et T)
- genèse possible d'anticorps facilitants in vivo (réponse B).

Il serait donc souhaitable de disposer d'antigènes permettant d'obtenir des anticorps suffisamment protecteurs vis-à-vis des cytokines, et limitant leur activité.

WO-A-98/51705 décrit des peptides de h RANTES, MIP1α et MIP1β compris entre la deuxième et la troisième cystéine de ces chimiokines qui se lient au co-récepteur CCR5.

WO-A-98/34631 décrit des peptides issus de chaînes γ de récepteurs de cytokines utilisés pour bloquer la fixation de la cytokine ou l'activation du récepteur.

WO-A-94/01457 décrit des peptides de l'IFN α utilisés comme substances porteuses de composés pharmaceutiques.

EP-A-0218531 décrit des peptides d'IL1 utilisés pour la préparation d'anticorps.

C'est pourquoi la présente demande a pour objet des peptides de taille comprise entre 5 et 40 acides aminés, provenant d'une cytokine, **caractérisés en ce que** au moins un de ses acides aminés et de préférence au moins deux de ses acides aminés consécutifs comportent au moins un de ses atomes espacé d'une distance d de moins de 5 angströms d'un atome du récepteur correspondant à ladite cytokine, l'espacement d étant évalué sur des données structurales (par exemple cristallographie ou RMN) et de préférence en ce qu'ils induisent des anticorps interagissant avec ladite cytokine, à l'exception
- des peptides compris entre la 2è et la 3è cystéine de h RANTES, de MIP 1α et de MIP 1β, et
- des peptides compris entre les acides aminés 123 et 140 de l'IFN α.

A cause de leur longueur, les peptides de cytokine selon l'invention correspondent à un nombre limité d'épitopes de la cytokine, en général à un ou deux épitopes de la cytokine, et sont en conséquence dépourvus des nombreux autres épitopes qu'elles renferment.

Par "cytokine", on entend aussi bien les cytokines vraies que les cytokines chimio-attractives encore appelées chimiokines. On préfère les cytokines humaines. Par "interagissant" on signifie que ces anticorps, pae exemple soit parce qu'ils reconnaissent la protéine native comme on peut le montrer par un test immunologique (ELISA, Western Blot), soit parce qu'ils bloquent la liaison de la cytokine à son récepteur comme on peut le montrer par un test d'activité biologique ou un test de compétition biochimique, ont un effet clinique bénéfique in vivo.

Parmi les cytokines, on peut citer par exemple le TGF β, l'IL1 α, l'IL1 β, le vEGF, le TNF α, les IFN α et γ, les IL 4,5,6,10,12,13,15,18,23, l'IP10, les MIP 1α et 1β, MCP1, et Rantes. Parmi les cytokines ci-dessus, on préfère le TGF β, l'IL1 β, le vEGF, le TNF α, les IFN α et γ, les IL 4,5,6,10,12,13,15,23 ou toute combinaison de certaines de ces cytokines et tout particulièrement l'IL1 β, le vEGF, le TNF α, l'IL23 et l'IFN α ou toute combinaison de certaines de ces cytokines.

Les peptides de cytokine selon l'invention proviennent ou dérivent d'une cytokine. Par "proviennent" l'on entend que leur séquence d'acides aminés est identique à celle de la cytokine. Par "dérivent" l'on entend que leur séquence d'acides aminés est majoritairement identique à celle de la cytokine mais comporte quelques différences comme on le verra ci-après.

Au moins un acide aminé des peptides de cytokine de l'invention et de préférence deux acides aminés consécutifs possède(nt) un de ses atomes espacé de moins de 5 angströms d'un atome du récepteur correspondant à ladite cytokine. Il est avantageusement espacé de moins de 4,5 angströms, notamment espacé de moins de 4 angströms et particulièrement espacé de moins de 3,5 angströms d'un atome du récepteur correspondant à ladite cytokine.

En général l'atome concerné de l'acide aminé se trouve sur la chaîne latérale dudit acide aminé.

Dans des conditions préférentielles de mise en oeuvre de l'invention 2, notamment 3 et de préférence 4 acides aminés consécutifs du peptide de cytokine répondent à ce même critère d'espacement.

Cet espacement est évalué sur des données structurales par exemple de cristallographie, ou encore par RMN qui donne des résultats similaires aux mesures cristallographiques.

Les peptides de cytokine ci-dessus comportent avantageusement plus de 8, notamment plus de 10, particulièrement plus de 12 et tout particulièrement plus de 15 acides aminés.

Dans d'autres conditions préférentielles de mise en oeuvre de l'invention, les peptides de cytokine ci-dessus comportent moins de 35, avantageusement moins de 30, notamment moins de 25 et particulièrement moins de 20 acides aminés. Généralement les séquences les plus courtes correspondent à des peptides contenant un seul groupe d'au moins deux acides aminés consécutifs comportant au moins un de leurs atomes espacé de moins de 5 angströms d'un atome du récepteur correspondant à ladite cytokine, tandis que les séquences les plus longues correspondent en général à des peptides selon l'invention contenant deux ou même trois groupes ou plus de tels acides aminés consécutifs. En effet ces groupes peuvent être espacés par plusieurs, par exemple 10 acides aminés comme dans le cas de l'IL1β.

Parmi les peptides de cytokine ci-dessus, on retient notamment un ou plusieurs peptides choisis parmi ou provenant de ceux dont les noms suivent:

| | |
|---|---|
| - hIL1β (Human Interleukin 1 beta) | |
| | 1-APVRSLNCTL-10 (ID SEQ N° 1) |
| | 29-LHLQGQDMEQQ-39 (ID SEQ N° 2) |
| | 123-STSQAENMPV-132 (ID SEQ N° 3) |
| - hvEGF (Human vascular Endothelial Growth Factor) | |
| | 73-IMRIKPHQGQHIGEMS-88 (ID SEQ N° 4) |
| - hTNFα (Human Tumor Necrosis Factor alpha) | |
| | 20-PQAEGQLQWLNRRANALLANGVELRDNQLVVPSEG-54 |
| | (ID SEQ N° 5) |
| | 80-ISRIAVSYQTKVNLLS-95 (ID SEQ N° 6) |
| | 124-FQLEKGDRLSAEINR-138 (ID SEQ N° 7) |
| - hIFNγ (Human Interferon gamma) | |
| | 1-MQDPYVKEAENLKKYFNAGHSDVADNGTLFLGILKN-36 |
| | (ID SEQ N° 8) |
| | 118-MAELSPAAKTGKRKRS-133 (ID SEQ N° 9) |
| - hIL10 (Human Interleukin 10) | |
| | 20-PNMLRDLRDAFSRVKTFFQMKDQLDNLLLKE-50 (ID SEQN° 10) |
| | |
| - hIL4 (Human Interleukin 4) | |
| | 5-ITLQEIIKTLNSL-17 (ID SEQ N° 11) |
| | 70-AQQFHRHKQLIRFLKRLDRNLWGLAG-95 (ID SEQ N° 12) |
| - hIL12p40 (Human Interleukin 12 sous unité p40) | |
| | 80-LLLHKKEDGIWSTDILKDQKEPKNKTFLRCE-110 |
| | (ID SEQ N° 13) |
| | 135-KSSRGSSDPQG-145 (ID SEQ N° 14) |
| - hIL18 (Human Interleukin 18) | |
| | 1-YFGKLESKLSVIRNLNDQVLFIDQGNRPLFEDMTD-35 |
| | (ID SEQ N° 15) |
| | 68-CEKISTLSCEN-78 (ID SEQ N° 16) |
| | 141-EDELGDRSIMFTVQNED-157 (ID SEQ N° 17) |
| - hIP10 (Human Interferon gamma inducible protein) | |
| | 39-VEIIATMKKKGEKRCLNPESKA-60 (ID SEQ N° 18) |
| - hIL5 (Human Interleukin 5) | |
| | 1-IPTSALVKETLALLSTHRTLLIANET-26 (ID SEQ N° 19) |
| | 96-LQEFLGVMNTEWI-108 (ID SEQ N° 20) |
| - hTGFp2 (Human Transforming Growth Factor beta type 2) | |
| | 25-KRDLGWKWIHE-35 (ID SEQ N° 21) |
| | 87-TILYYIGKTPKIEQ -100 (ID SEQ N° 22) |
| - hIL15 (Human Interleukin 15) | |
| | 1-ANWVNVISDLKKI-13 (ID SEQ N° 23) |
| | 74- SSNGNVTESGCKECEELEKKNIKEFLQSFVHIVQMF -111 (ID SEQ N° 24) |
| - hIL6 (Human Inlerleukin 6) | |
| | 114-RAVQMSTKVLIQFLQKKAKNLDAITTPDPTTNASLL-149 (ID SEQ N° 26) |
| | 28-KQIRYILDGISA-39 (ID SEQ N° 25) |
| - hMIP1α (Human Macrophage Inflammatory Protein alpha) | |
| | 51-ADPSEEWVQKYVSDLELSA-69 (ID SEQ N° 27) |
| - hMIP1β (Human Macrophage Inflammatory Protein beta) | |
| | 52-ADPSESWVQEYVYDLELN-69 (ID SEQ N° 28) |
| - hIL13 (Human Interleukin 13) | |
| | 8-TALRELIEEL-17 (ID SEQ N° 29) |
| | 57-CSAIEKTQRMLSGFCPHKVSAGQFSS-82 (ID SEQ N° 30) |
| - hIL23 (Human Interleukin 23) | |
| | 52 GHMDLREEGDEETT 65 (ID SEQ N° 31) |
| | 115 LLPDSPVGQLHASLLGLSQ 133 (ID SEQ N° 32) |
| | 160 LLRFKILRSLQAFVAVAARV 179 (ID SEQ N° 33) |
| - hRANTES (Human Regulated upon Activation Normal T-cell expressed) | |
| | 51-ANPEKKWVREYINSLEMS-68 (ID SEQ N° 34) |
| -hIFNα (Human Interferon alpha) | |
| | 12-RRTLMLLAQMRK-23 (ID SEQ N° 35) |
| | 95-LEACVIQGVGVTETPLMKEDSILAVRK-121 (ID SEQ N° 36) |

Les séquences qui précèdent ont été choisies dans les cytokines qui participent au développement de maladies humaines de par leur rôle dans la réponse inflammatoire ou la réponse immunitaire spécifique.

On retient tout particulièrement les peptides suivants :
- 123-STSQAENMPV-132 de hIL1β
- 73-IMRIKPHQGQHIGEMS de hvEGF
- 20-PQAEGQLQWLNRRANALLANGVELRDNQLWPSEG-54 de hTNFα
- 1-MQDPYVKEAENLKKYFNAGHSDVADNGTLFLGILKN-36 de hIFNγ
- 20-PNMLRDLRDAFSRVKTFFQMKDQLDNLLLKE-50 de hIL10
- 70-AQQFHRHKQLIRFLKRLDRNLWGLAG-95 de hIL4
- 1-YFGKLESKLSVIRNLNDQVLFIDQGNRPLFEDMTD-35 de hIL18
- 52 GHMDLREEGDEETT 65,115 LLPDSPVGQLHASLLGLSQ 133 ou 160 LLRFKILRSLQAFVAVAARV 179 de hIL23.

Comme le sait l'homme de l'art de l'immunologie, des modifications des enchaînements peptidiques naturels sont possibles sans pour autant modifier la nature des propriétés immunologiques des peptides immunogènes. On peut donc citer également les dérivés de peptides de cytokine qui sont fortement homologues à ces séquences naturelles, par exemple ayant plus de 50% d'homologie, en particulier plus de 70% d'homologie, et de préférence plus de 80% d'homologie ou même plus de 90% d'homologie avec le peptide natif correspondant tout en conservant les propriétés immunologiques de ce site épitopique du peptide natif. Leur zone d'homologie peut varier de 5 à 40 résidus, par exemple de 8 à 40 résidus, ou encore de 8 à 35 résidus, de préférence de 10 à 35 résidus mais aussi de 12 à 35 résidus, notamment de 12 à 30 résidus, en particulier de 15 à 30 résidus et tout particulièrement de 15 à 25 résidus.

Les dérivés des peptides de cytokine peuvent contenir des résidus modifiés, à condition que les modifications n'abaissent pas sensiblement l'immunogénicité, soit par addition de radicaux chimiques (méthyle, acétyle etc...) soit par modification stéréochimique (utilisation d'acides aminés de série D). Les dérivés peptidiques de cytokine doivent, comme les peptides de cytokine induire des anticorps interagissant avec la cytokine.

Les dérivés peptidiques de cytokine selon l'invention peuvent comporter une ou plusieurs modifications dans les acides aminés dont ils sont constitués telles que des délétions, substitutions, additions, ou fonctionnalisations (telles qu'acylation) d'un ou plusieurs acides aminés, dans la mesure où ces modifications restent dans le cadre précisé ci-dessus (caractères immunologiques). Par exemple, en général le remplacement d'un résidu leucine par un résidu isoleucine ne modifie pas de telles propriétés ; les modifications doivent généralement concerner moins de 40% d'acides aminés, notamment moins de 30% de préférence moins de 20% et tout particulièrement moins de 10% des acides aminés du peptide naturel. Il est important que les anticorps induits par les peptides modifiés soient actifs vis à vis de la cytokine native.

Ces modifications sont à la portée de l'homme de l'art qui peut vérifier l'incidence des modifications par des tests simples. L'immunogénicité de tels dérivés modifiés peut être évaluée par ELISA après immunisation de souris, l'antigène testé par ELISA étant la cytokine entière ou le peptide de cytokine immunisant, ou par des tests de blocage de la liaison cytokine-récepteur. Les éventuelles modifications affectent de préférence moins de 8 acides aminés, avantageusement moins de 6 acides aminés, notamment moins de 4 acides aminés, et particulièrement 3 acides aminés ou moins comme 2 ou 1 seul acide aminé.

L'invention a encore pour objet un composé caractérisé en ce qu'il renferme au moins un peptide de cytokine ou dérivé de peptide de cytokine ci-dessus. Un tel composé peut comprendre des répétitions de peptides/dérivés identiques, ou des combinaisons de peptides/dérivés différents, que ce soit sous forme linéaire ou sous forme de structure en candélabre ou de couplages mixtes à des protéines porteuses. Un tel composé peut également se présenter sous forme cyclisée. Ainsi des peptides de cytokine ou les dérivés peptidiques de cytokine selon l'invention peuvent par exemple être insérés dans des séquences plus longues d'acides aminés donnant notamment une meilleure conformation ou combinés à des épitopes T exogènes (que ce soit pour des immunisations protéiques ou par ADN).

Ils peuvent avantageusement être associés de manière covalente à des protéines porteuses comme par exemple le KLH.

Comme on l'a vu, les peptides de cytokine selon l'invention correspondent en général à un petit nombre d'épitopes de la cytokine. Lorsqu'ils sont notamment insérés, combinés ou associés, les composés ci-dessus ne comportent pas d'autres épitopes de ladite cytokine.

Ces peptides de cytokine ou dérivés de cytokine pourront être inclus dans toute séquence de protéine qui ne comprendra pas d'homologie avec les autres épitopes de la cytokine naturelle. Par exemple, ils pourront être les sites de liaison au récepteur, aux extrémités desquelles on ajoute simplement une cystéine pour conférer au peptide une structure cyclique. Un autre exemple est un peptide entouré de séquences d'épitopes T de la toxine tétanique. Encore un autre exemple pourra comprendre un peptide correspondant à la séquence du site de liaison au récepteur mais où certains acides aminés sont remplacés par leurs isomères de série D afin d'éviter leur effet agoniste. En effet, il pourra être éventuellement avantageux d'utiliser des dérivés peptidiques qui n'ont pas d'activité agoniste sur le récepteur afin que l'immunogène ne puisse pas interférer avec la réponse immunitaire.

De façon à augmenter la réponse immunitaire, ces peptides de cytokine ou dérivés de cytokine pourront être couplés à des protéines porteuses. Les méthodes de couplages et la protéine porteuse considérées pourront être différentes selon le peptide ciblé : il pourra s'agir par exemple de la protéine Keyhole Limpet Hemocyanin (KLH) et Tetanus Toxoid (TT) conjugués aux peptides par des méthodes chimiques bien connues de l'homme de l'art comme celles de couplage par le carbodiimide, ou par le glutaraldéhyde ou par la benzidine bis-diazotée. La réalisation de ces couplages pourra être facilitée par l'addition ou l'incorporation d'acides aminés à la séquence comme par exemple des lysines, des histidines, des tyrosines ou des cystéines. De tels composés peptidiques couplés à un épitope T exogène (provenant de plasmodium falciparum, de KLH, etc..) que ce soit de manière chimique ou de manière génétique entrent aussi dans le cadre de l'invention.

Des couplages en réseau de type en candélabre ou à des molécules telles que la transferrine ou la ferritine peuvent être aussi mis en oeuvre pour stimuler efficacement la réponse immunitaire.

Les peptides selon l'invention peuvent être notamment produits par synthèse chimique ou par génie génétique ou par tout autre méthode adaptée. La synthèse de peptides cycliques, au besoin en greffant un ou plusieurs acides aminés en bout de chaîne comme des cystéines pour créer un pont disulfure permet de retrouver une partie de la structure secondaire que ces fragments peptidiques possèdent dans la structure tridimensionnelle de la protéine.

Les peptides de cytokine, dérivés de cytokine et leurs composés selon l'invention possèdent de très intéressantes propriétés pharmacologiques. Ils sont doués notamment de remarquables propriétés anti-cytokines. Ils sont en effet immunogènes et capables de générer chez un sujet des anticorps reconnaissant la cytokine native. Ces peptides ne contiennent pas les nombreux autres épitopes provenant de la cytokine à laquelle ils correspondent.

Ces propriétés sont illustrées ci-après dans la partie expérimentale. Elles justifient l'utilisation des peptides ci-dessus décrits à titre de médicament.

Le fait de se limiter à ces peptides proches du site de liaison au récepteur, à l'exclusion des autres épitopes des cytokines, donne notamment l'avantage de limiter la génération d'anticorps facilitants ou potentialisateurs de l'activité cytokinique. De plus, ils permettent d'augmenter la qualité de l'immunisation anticytokine car on limite le nombre de déterminants antigéniques ciblés.

C'est pourquoi l'invention a encore pour objet des médicaments **caractérisés en ce qu**'ils sont constitués des peptides de cytokine ou dérivés de cytokine ou composés tels que définis ci-dessus, c'est à dire des peptides de cytokine ou dérivés de cytokine ou composés immunogènes tels que définis ci-dessus, ou compris entre la 2è et la 3è cystéine de h RANTES ou compris entre les acides aminés 123 et 140 de l'IFN α, pour leur utilisation dans une méthode de traitement thérapeutique du corps humain ou animal, ainsi que l'utilisation d'un tel peptide de cytokine ou dérivé de cytokine ou composé immunogène pour la préparation d'un médicament curatif ou préventif destiné au traitement ou à la prévention des affections liées à un excès ou à la présence de cytokines.

Les médicaments selon la présente invention trouvent leur emploi par exemple dans le traitement tant curatif que préventif des maladies liées aux dérèglements du système immunitaire impliquant des surproductions de cytokines comme par exemple les maladies auto-immunes (comprenant entre autres la sclérose en plaques, la polyarthrite rhumatoïde, le psoriasis, le diabète auto-immun, le lupus), l'allergie ou l'asthme, les cancers ou le SIDA. Ainsi il est clair que la lutte contre l'IL1β ou le TNFα peut-être utile dans la polyarthrite rhumatoïde, la lutte contre l'IFNγ, l'IL18, l'IL23 ou l'IL12 peut être utile dans la lutte contre la sclérose en plaques, ou le diabète auto-immun, la lutte contre l'IL4, l'IL5 et l'IL13 peut être utile contre l'allergie ou l'asthme, la lutte contre l'IL10 ou le vEGF peut être utile contre certains cancers. Plus généralement les dérèglements Th1/Th2 qui gouvernent la réponse immunitaire elle-même et qui font classiquement intervenir IL12, IL2, IL4, IL6, IL10, IL13, IFNγ, TNFα pourront bénéficier d'un réajustement de l'équilibre par l'immunisation active. Ce ne sont là que quelques exemples, et l'invention a aussi pour objet tout traitement du corps humain, basé sur une immunisation active (ADN ou peptide) impliquant les séquences peptidiques mentionnées ci-dessus à l'exclusion des autres épitopes des cytokines. Ces séquences pourront être modifiées comme indiqué dans la présente description, et les immunisations par ADN se feront par simple traduction à partir du code génétique.

La réponse immunitaire humorale pourra être évaluée par des tests ELISA ou des tests montrant l'inhibition de la liaison de la cytokine native à son récepteur. La réponse cellulaire pourra être évaluée en présence de tests de prolifération cellulaire face au peptide utilisé.

Les principes actifs immunogènes selon l'invention peuvent être utilisés comme suit :

On administre à un patient un peptide de cytokine ou dérivé de cytokine ou composé immunogène selon la présente invention, par exemple par voie sous-cutanée ou intramusculaire, en quantité suffisante pour être efficace sur le plan thérapeutique, à un sujet ayant besoin d'un tel traitement. La dose administrée peut aller par exemple de 1 à 1000 µg, notamment 10 à 500 µg par voie sous-cutanée, une fois par mois pendant trois mois, puis périodiquement en fonction du taux des anticorps sériques induits, par exemple tous les 2-6 mois. On pourra administrer dans une même préparation deux ou plusieurs molécules immunogènes différentes pour induire des anticorps neutralisant toutes les sites fonctionnels délétères au cas où une seule molécule immunogène ne porte pas tous les sites actifs de la cytokine surproduite que l'on veut neutraliser.

L'invention a aussi pour objet les compositions pharmaceutiques notamment les vaccins qui renferment au moins un peptide de cytokine ou dérivé de cytokine ou composé immunogène précité, à titre de principe actif.

A titre de médicaments, un peptide de cytokine ou dérivé de cytokine ou composé immunogène de l'invention peut être incorporé dans des compositions pharmaceutiques destinées à n'importe quelle voie conventionnelle en usage dans le domaine des vaccins, en particulier par voie sous-cutanée, par voie intramusculaire, par voie intraveineuse ou par voie orale. L'administration peut avoir lieu en dose unique ou répétée une ou plusieurs fois après un certain intervalle de temps.

C'est pourquoi la présente demande a également pour objet une composition pharmaceutique, curative ou préventive, caractérisée en ce qu'elle comprend à titre de principe actif, un ou plusieurs peptides de cytokine ou dérivés de cytokine ou composés immunogènes nouveaux ou compris entre les acides aminés 123 et 140 de l'IFN α, tel que définis ci-dessus.

L'agent immunogène peut être conditionné seul ou mélangé à un excipient ou mélange d'excipients pharmaceutiquement acceptables tel qu'un adjuvant. La présente demande a plus particulièrement pour objet un vaccin contenant à titre d'immunogène, un peptide de cytokine ou dérivé de cytokine ou composé immunogène ci-dessus.

La présente invention a encore pour objet un procédé de préparation d'une composition ci-dessus décrite, caractérisé en ce que l'on mélange, selon des méthodes connues en elles mêmes, le ou les principes actifs avec des excipients acceptables, notamment pharmaceutiquement acceptables.

L'administration à un patient d'un un peptide de cytokine ou dérivé de cytokine ou composé immunogène selon l'invention correspond à une immunothérapie active. Il peut être intéressant également de procéder à une immunothérapie passive, c'est à dire de fournir à un patient directement des anticorps dont il a besoin.

Les préparations vaccinales pourront être conditionnées pour la voie intra nasale sous forme de gel avec comme excipient le carbopol, de gouttes nasales ou de spray et pour la voie orale sous forme de capsules gastrorésistantes, de dragées ou de granules gastrorésistants.

Dans le cas de vaccin ADN administré par voie systémique ou mucosale, la présentation galénique du plasmide pourra être une suspension dans un liquide physiologique tel le PBS physiologiques (tampon phosphate = PBS). Les plasmides pourront être inclus dans des microsphères de polymères biodégradable (PLG, PLA, PCL) et administrées dans des capsules gastrorésistantes pour ingestion (voie orale). L'ADN pourra également être exprimé dans un vecteur vivant bactérien, type salmonelle ou viral type adénovirus ou poxvirus.

La présente demande a enfin pour objet un procédé d'immunisation active de patients caractérisé en ce que l'on utilise à titre d'immunogène un peptide de cytokine ou dérivé de cytokine ou composé immunogène tel que défini ci-dessus avantageusement associé à un adjuvant d'immunité minéral, huileux ou de synthèse.

Les immunisations pourront se faire classiquement notamment par des peptides ou des composés immunogènes comme des conjugués de préférence en présence d'un adjuvant, par exemple l'ISA 51 ou l'Alun. Les immunisations pourront se faire à base d'ADN (séquences homologues aux sites de liaison combinées avec des épitopes T exogènes) en utilisant de l'ADN nu ou un vecteur d'expression contenant un promoteur adapté comme par exemple le pCR3.1. Les ADN administrés pourront être protégés des nucléases par l'utilisation de radicaux adéquats (CpG etc..). On pourra notamment faire suivre une immunisation initiale par ADN par des rappels classiques à l'aide des composés peptidiques.

Les conditions préférentielles de mise en oeuvre des peptides ci-dessus décrits s'appliquent également aux autres objets de l'invention visés ci-dessus.

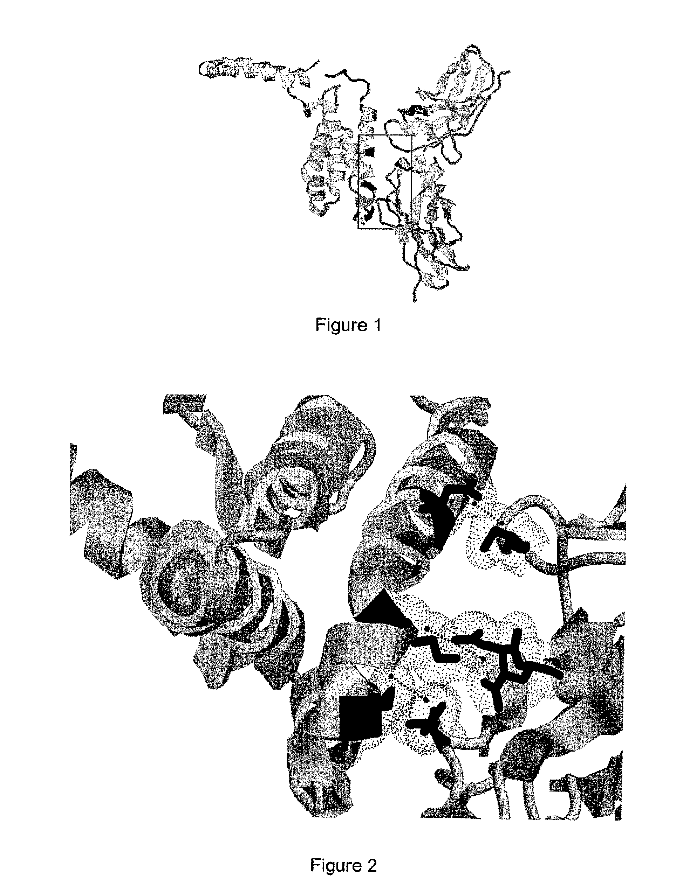

La figure 1 et la figure 2 qui est un agrandissement de la figure 1 représentent la structure cristallographique du peptide provenant de l'IL10 en contact avec son récepteur. On distingue la cytokine IL10 (à gauche) en contact avec son récepteur (à droite).

Sur la figure 2, on peut observer que les acides aminés marqués en noir sont espacés de moins de 4 Angströms (d=pointillé entre deux boules noires). Ils correspondent de bas en haut aux couples :
1921L10R Sérine vis-à-vis du 28IL10 Acide Aspartique (3.18Å) , 100IL10R Acide aspartique et 101IL10R acide glutamique vis-à-vis du 34IL10 Lysine (3.83A et 3.84A), et 94IL10R Asparagine vis-à-vis du 39IL10 Méthionine (3.70Å). Pour chaque acide aminé un point central a été déterminé comme étant le barycentre du carbone alpha de l'acide aminé considéré, de l'extrémité de la chaîne latérale et d'un troisième point choisi comme étant le plus extrême des deux précédents sur la chaîne latérale. La distance d mesurée est la distance séparant les points centraux des acides aminés respectivement de la cytokine et de son récepteur. On peut évidemment définir les distances en utilisant d'autres méthodes usuelles.

Les exemples qui suivent illustrent la présente invention.

### Exemple 1 :

Le peptide KLHLQGQDMEQQ (ID SEQ N° 37) a été synthétisé à partir de la séquence d'IL1β humaine, le résidu K a été ajouté à la séquence naturelle pour permettre une liaison au KLH par couplage à l'aide de glutaraldéhyde.
Cinq souris ont été immunisées en présence de l'adjuvant ISA51. Pour la première injection réalisée à J0, on prépare une émulsion de 40µg d'immunogène dans une quantité d'ISA51 suffisante pour préparer 100µl d'émulsion. Pour le rappel à J21 et à J40, on prépare 100µL d'émulsion contenant 20µg d'immunogène. Cinq souris ont été immunisées en parallèle comme contrôles avec du KLH dans le même adjuvant.

A J60, du sérum est prélevé et des tests ELISA sont réalisés avec la cytokine native pour révéler la présence d'anticorps.

L'absorbance moyenne obtenue pour chaque souris est présentée dans le tableau ci-dessous :

| Souris | 1 | 2 | 3 | 4 | 5 | Moyenne |
|---|---|---|---|---|---|---|
| Peptide | 1.34 | 0.08 | 1.86 | 0.90 | 1.56 | 1.15 |
| Contrôle | 0.05 | 0.11 | 0.08 | 0.12 | 0.06 | 0.08 |

L'existence d'anticorps neutralisant la cytokine native a pu être évaluée de la façon suivante : 20 unités de cytokine IL1β native sont préincubées avec du sérum de souris (immunisée ou contrôle) dilué du 1/100^{ième} au 1/2000^{ième} pendant 2H à 37 °C. Le tout est ensuite ajouté à des cellules EL4, mises à 50 000 cellules en micropuits. La production d'IL2 est évaluée après 24 heures dans le surnageant par un test ELISA sandwich (R&D diagnostics). Le pourcentage d'inhibition de la production d'IL2 indique le pourcentage de neutralisation de l'IL1β. Comme on peut le voir, on observe des réponses pour les souris immunisées mais pas pour les contrôles.

| Souris | 1 | 2 | 3 | 4 | 5 | Moyenne |
|---|---|---|---|---|---|---|
| Peptide | 52% | 25% | 93% | 86% | 8% | 52% |
| Contrôle | 8% | -5% | 11% | 5% | 12% | 6% |

### Exemple 2 :

On a immunisé 5 souris avec le peptide de synthèse provenant du vEGF humain : KPHQGQHIGEMS. (ID SEQ N° 38) Ce peptide a été couplé à la protéine porteuse KLH par réaction au glutaraldéhyde. Après immunisation dans les mêmes conditions que dans l'exemple 1, du sérum est prélevé à J60 et des tests ELISA sont réalisés avec la cytokine native pour révéler la présence d'anticorps.

L'absorbance moyenne obtenue pour chaque souris est présentée dans le tableau ci-dessous :

| Souris | 1 | 2 | 3 | 4 | 5 | Moyenne |
|---|---|---|---|---|---|---|
| Peptide | 1.50 | 1.00 | 0.86 | 0.98 | 1.63 | 1.19 |
| Contrôle | 0.05 | 0.13 | 0.07 | 0.10 | 0.08 | 0.09 |

### Exemple 3 :

On a immunisé 5 souris avec le peptide de synthèse provenant du TNFα humain qui est: KYQAEGQLQWLNRRANALLANGVELRDNQL. (ID SEQ N° 39) Ce peptide a été couplé à la protéine porteuse KLH par réaction au diazobenzidine. Les résidus K et Y ont été ajoutés à la séquence naturelle pour permettre une liaison au KLH par couplage à l'aide de glutaraldéhyde. Après immunisation dans les mêmes conditions que dans l'exemple 1, du sérum est prélevé à J60 et des tests ELISA sont réalisés avec la cytokine native pour révéler la présence d'anticorps.

L'absorbance moyenne obtenue pour chaque souris est présentée dans le tableau ci-dessous :

| Souris | 1 | 2 | 3 | 4 | 5 | Moyenne |
|---|---|---|---|---|---|---|
| Peptide | 1.64 | 1.14 | 1.96 | 1.02 | 0.78 | 1.31 |
| Contrôle | 0.11 | 0.16 | 0.09 | 0.11 | 0.18 | 0.13 |

L'existence d'anticorps neutralisant la cytokine native a pu être évaluée de la façon suivante : 50 unités de cytokine TNFα native sont préincubées avec du sérum de souris (immunisée ou contrôle) dilué au 1/200^{ième} pendant 2H à 37 °C. Le tout est ensuite ajouté à des cellules L929, mise à 25 000 cellules par micropuits. La lyse des cellules est évaluée après 24 heures par coloration au naphtol blue black (Sigma). Le pourcentage d'inhibition de la lyse indique le pourcentage de neutralisation du TNFα, et comme on peut voir, on observe des réponses pour les souris immunisées mais pas les contrôles.

| Souris | 1 | 2 | 3 | 4 | 5 | Moyenne |
|---|---|---|---|---|---|---|
| Peptide | 88% | 12% | 93% | 36% | 79% | 61% |
| Contrôle | 5% | 6% | 14% | 5% | 1% | 6% |

### Exemple 4 :

On a immunisé 5 souris avec le peptide provenant de l'IFNγ humain qui est: KKYFNAGHSDVADNGTLFLGILKN (ID SEQ N° 40). Ce peptide a été synthétisé chimiquement sous forme de MAPS. Ce peptide MAPS a ensuite été couplé à la protéine porteuse Tétanus Toxoid par réaction au glutaraldéhyde. Après immunisation dans les mêmes conditions que dans l'exemple 1, du sérum est prélevé à J60 et des tests ELISA sont réalisés avec la cytokine native pour révéler la présence d'anticorps.

L'absorbance moyenne obtenue pour chaque souris est présentée dans le tableau ci-dessous :

| Souris | 1 | 2 | 3 | 4 | 5 | Moyenne |
|---|---|---|---|---|---|---|
| Peptide | 1.56 | 1.05 | 0.98 | 1.80 | 1.64 | 1.41 |
| Contrôle | 0.10 | 0.12 | 0.23 | 0.08 | 0.09 | 0.12 |

L'existence d'anticorps neutralisant la cytokine native a pu être évaluée de la façon suivante : 100 unités de cytokine native sont pré incubées avec du sérum de souris (immunisée ou contrôle) dilué du 1/250^{ième} pendant 2H à 37 °C. Le tout est ensuite ajouté à des cellules RAW 264.7, mises à 300 000 cellules dans des puits de 2 ml. L'expression de CMH de classe Il sur les cellules est évaluée par cytométrie de flux après 24 heures, par marquage avec des anticorps anti-CMH de classe ll couplés à la fluorescéine. Le pourcentage d'inhibition de l'expression du CMH de classe ll indique le pourcentage de neutralisation de l'IFNγ, et comme on peut voir, on observe des inhibitions pour les souris immunisées mais pas les contrôles. Le seuil de signification pour ces expériences est de 30%.

| Souris | 1 | 2 | 3 | 4 | 5 | Moyenne |
|---|---|---|---|---|---|---|
| Peptide | 40% | 89% | 67% | 38% | 83% | 63% |
| Contrôle | 25% | 14% | -10% | 29% | 7% | 13% |

### Exemple 5 :

On a immunisé 5 souris avec le peptide provenant de l'IL10 humaine qui est: DECNMLRDLRDAFSRVKTFFQMKDQLDNC (ID SEQ N° 41). Ce peptide a été synthétisé chimiquement, et une cystéine a été ajoutée à chaque extrémité de sorte à en faire un peptide cyclique. Ce peptide a ensuite été couplé à la protéine porteuse KLH par réaction au carbodiimide. Le résidu DE a aussi été ajouté à la séquence naturelle pour permettre une liaison au KLH par réaction au carbodiimide. Après immunisation dans les mêmes conditions que dans l'exemple 1, du sérum est prélevé à J60 et des tests ELISA sont réalisés avec la cytokine native pour révéler la présence d'anticorps.

L'absorbance moyenne obtenue pour chaque souris est présentée dans le tableau ci-dessous :

| Souris | 1 | 2 | 3 | 4 | 5 | Moyenne |
|---|---|---|---|---|---|---|
| Peptide | 1.94 | 0.96 | 1.85 | 0.97 | 1.5 | 1.44 |
| Contrôle | 0.29 | 0.52 | 0.17 | 0.26 | 0.36 | 0.32 |

### Exemple 6 :

On a immunisé 5 souris avec le peptide correspondant à l'IL4 humaine qui est : KQLIRFLKRLDRNLWGLAG (ID SEQ N° 42). Ce peptide a été couplé à la protéine porteuse KLH par réaction au glutaraldéhyde. Après immunisation dans les mêmes conditions que dans l'exemple 1, du sérum est prélevé à J60 et des tests ELISA sont réalisés avec la cytokine native pour révéler la présence d'anticorps.

L'absorbance moyenne obtenue pour chaque souris est présentée dans le tableau ci-dessous :

| Souris | 1 | 2 | 3 | 4 | 5 | Moyenne |
|---|---|---|---|---|---|---|
| Peptide | 1.06 | 1.25 | 0.95 | 0.87 | 1.24 | 1.07 |
| Contrôle | 0.34 | 0.09 | 0.26 | 0.16 | 0.12 | 0.19 |

### Exemple 7 :

On a immunisé 5 souris avec le peptide correspondant à l'IL12p40 humaine qui est: KKEDGIWSTDILKDQKEPKNKTFLRCE (ID SEQ N° 43). Ce peptide a été couplé à la protéine porteuse Tetanos Toxoïde par réaction au benzidine bis-diazoté (diazo). Après immunisation dans les mêmes conditions que dans l'exemple 1, du sérum est prélevé à J60 et des tests ELISA sont réalisés avec la cytokine native pour révéler la présence d'anticorps.

L'absorbance moyenne obtenue pour chaque souris est présentée dans le tableau ci-dessous :

| Souris | 1 | 2 | 3 | 4 | 5 | Moyenne |
|---|---|---|---|---|---|---|
| Peptide | 0.95 | 1.06 | 1.58 | 1.14 | 0.87 | 1.12 |
| Contrôle | 0.06 | 0.12 | 0.09 | 0.05 | 0.09 | 0.08 |

### Exemple 8 :

On a immunisé 5 souris avec le peptide provenant de l'IL18 humaine qui est: KYFGKLESKLSVIRNLNDQVLFID (ID SEQ N° 44). Ce peptide a été couplé à la protéine porteuse KLH par réaction au glutaraldéhyde. Le résidu K a été ajouté à la séquence naturelle pour permettre une liaison au KLH par couplage à l'aide de glutaraldéhyde. Après immunisation dans les mêmes conditions que dans l'exemple 1, du sérum est prélevé à J60 et des tests ELISA sont réalisés avec la cytokine native pour révéler la présence d'anticorps.

L'absorbance moyenne obtenue pour chaque souris est présentée dans le tableau ci-dessous :

| Souris | 1 | 2 | 3 | 4 | 5 | Moyenne |
|---|---|---|---|---|---|---|
| Peptide | 1.85 | 1.13 | 0.99 | 1.24 | 1.54 | 1.35 |
| Contrôle | 0.31 | 0.09 | 0.27 | 0.16 | 0.24 | 0.21 |

### Exemple 9 :

On a immunisé 5 souris avec le peptide synthétique provenant de l'IP10 humain qui est : KKKGEKRCLNPESKA (ID SEQ N° 45). Ce peptide a été couplé à la protéine porteuse Tetanos Toxoïde par réaction au glutaraldéhyde. Les trois résidus K présents dans la séquence naturelle permettent une liaison au KLH par couplage à l'aide de glutaraldéhyde. Après immunisation dans les mêmes conditions que dans l'exemple 1, du sérum est prélevé à J60 et des tests ELISA sont réalisés avec la cytokine native pour révéler la présence d'anticorps.

L'absorbance moyenne obtenue pour chaque souris est présentée dans le tableau ci-dessous :

| Souris | 1 | 2 | 3 | 4 | 5 | Moyenne |
|---|---|---|---|---|---|---|
| Peptide | 1.16 | 0.98 | 1.24 | 1.09 | 0.88 | 1.07 |
| Contrôle | 0.32 | 0.21 | 0.11 | 0.20 | 0.09 | 0.19 |

### Exemple 10 :

On a immunisé 5 souris avec le peptide correspondant à l'IL5 humaine qui est : KLQEFLGVMNTEWI (ID SEQ N° 46). Ce peptide a été couplé à la protéine porteuse KLH par réaction au glutaraldéhyde. Le résidu K a été ajouté à la séquence naturelle pour permettre une liaison au KLH par couplage à l'aide de glutaraldéhyde. Après immunisation dans les mêmes conditions que dans l'exemple 1, du sérum est prélevé à J60 et des tests ELISA sont réalisés avec la cytokine native pour révéler la présence d'anticorps.

L'absorbance moyenne obtenue pour chaque souris est présentée dans le tableau ci-dessous :

| Souris | 1 | 2 | 3 | 4 | 5 | Moyenne |
|---|---|---|---|---|---|---|
| Peptide | 1.25 | 1.21 | 1.87 | 1.10 | 0.88 | 1.26 |
| Contrôle | 0.51 | 0.21 | 0.42 | 0.09 | 0.38 | 0.32 |

### Exemple 11:

On a immunisé 5 souris avec le peptide correspondant à le TGFβ2 humain qui est : DTILYYIGKTPKIE (ID SEQ N° 47). Ce peptide a été couplé à la protéine porteuse KLH par réaction au carbodiimide. Le résidu D a été ajouté à la séquence naturelle pour permettre une liaison par couplage. Après immunisation dans les mêmes conditions que dans l'exemple 1, du sérum est prélevé à J60 et des tests ELISA sont réalisés avec la cytokine native pour révéler la présence d'anticorps.

L'absorbance moyenne obtenue pour chaque souris est présentée dans le tableau ci-dessous :

| Souris | 1 | 2 | 3 | 4 | 5 | Moyenne |
|---|---|---|---|---|---|---|
| Peptide | 0.95 | 1.15 | 0.99 | 1.17 | 1.08 | 1.07 |
| Contrôle | 0.40 | 0.09 | 0.35 | 0.26 | 0.29 | 0.28 |

### Exemple 12 :

On a immunisé 5 souris avec le peptide correspondant à l'IL6 humaine qui est : KQIRYILDGISA (ID SEQ N° 25). Ce peptide a été couplé à la protéine porteuse TT par réaction au benzidine bis-diazoté (diazo). Après immunisation dans les mêmes conditions que dans l'exemple 1, du sérum est prélevé à J60 et des tests ELISA sont réalisés avec la cytokine native pour révéler la présence d'anticorps.

L'absorbance moyenne obtenue pour chaque souris est présentée dans le tableau ci-dessous :

| Souris | 1 | 2 | 3 | 4 | 5 | Moyenne |
|---|---|---|---|---|---|---|
| Peptide | 1.11 | 1.27 | 1.02 | 1.34 | 0.32 | 1.01 |
| Contrôle | 0.11 | 0.15 | 0.29 | 0.14 | 0.21 | 0.21 |

### Exemple 13

Les ADN correspondants aux peptides 1-APVRSLNCTL-10 (GCACCTGTACGATCACTGAACTGCACGCTC) (ID SEQ N° 48), 29-LHLQGQDMEQQ-39 (CTCCACCTCCAGGGACAGGATATGGAGCAACAA) (ID SEQ N° 49) et 123-STSQAENMPV-132
(AGCACCTCTCAAGCAGAAAACATGCCCGTC) (ID SEQ N°50) de l'IL1β ont été synthétisés et sont séparés des épitopes T de la toxine tétanique : AQYIKANSKFIGITEL (ID SEQ N° 55)
(CAGTACATCAAGGCTAACTCCAAGTTCATCGGTATCACTGAGCTG) (ID SEQ N° 51)
et du KLH : VDTVVRKNVDSL
(GTTGACACCACCAGAAAAAATGTTGACTCCCTT) (ID SEQ N° 52), par des écarteurs GYG (GGCTACGGC) (ID SEQ N° 54).
La séquence nucléotidique finale est la suivante:

GTTGACACCACCAGAAAAAATGTTGACTCCCTTGGCTACGGCGCACCTG
TACGATCACTGAACTGCACGCTCGGCTACGGCGTTGACACCACCAGAAA
AAATGTTGACTCCCTTGGCTACGGCCTCCACCTCCAGGGACAGGATATG
GAGCAACAAGGCTACGGCCAGTACATCAAGGCTAACTCCAAGTTCATCG
GTATCACTGAGCTGGGCTACGGCAGCACCTCTCAAGCAGAAAACATGCC
CGTCGGCTACGGCGTTGACACCACCAGAAAAAATGTTGACTCCCTT
(ID SEQ N° 53).

Cette séquence d'ADN, clonée dans pRSET-A, code donc pour un polypeptide composé qu'on produit et purifie par génie génétique sous forme de protéine de fusion poly-Histidine. Ce polypeptide est utilisé comme immunogène comme dans l'exemple 1.

La réponse anticorps des souris est mesurée contre l'IL1β native par ELISA et on trouve les valeurs suivantes :

| Souris | 1 | 2 | 3 | 4 | 5 | Moyenne |
|---|---|---|---|---|---|---|
| peptide | 0.86 | 0.73 | 1.68 | 1.55 | 1.83 | 1.33 |
| Contrôle | 0.23 | 0.11 | 0.21 | 0.29 | 0.17 | 0.20 |

### Exemple 14.

Les ADNc correspondants aux peptides 1-APVRSLNCTL-10 (GCACCTGTACGATCACTGAACTGCACGCTC) (ID SEQ N° 48), 29-LHLQGQDMEQQ-39 (CTCCACCTCCAGGGACAGGATATGGAGCAACAA) (ID SEQ N° 49) et 123-STSQAENMPV-132
(AGCACCTCTCAAGCAGAAAACATGCCCGTC) (ID SEQ N° 50) de l'IL1β pont été synthétisés et sont séparés des épitopes T de la toxine tétanique AQYIKANSKFIGITEL
(CAGTACATCAAGGCTAACTCCAAGTTCATCGGTATCACTGAGCTG) (ID SEQ N° 51)
et du KLH :
VDTVVRKNVDSL (GTTGACACCACCAGAAAAAATGTTGACTCCCTT) (ID SEQ N° 52)
par des spacers GYG.
La séquence finale est la suivante: 
GTTGACACCACCAGAAAAAATGTTGACTCCCTTGGCTACGGCGCACCTG
TACGATCACTGAACTGCACGCTCGGCTACGGCGTTGACACCACCAGAAA
AAATGTTGACTCCCTTGGCTACGGCCTCCACCTCCAGGGACAGGATATG
GAGCAACAAGGCTACGGCCAGTACATCAAGGCTAACTCCAAGTTCATCG
GTATCACTGAGCTGGGCTACGGCAGCACCTCTCAAGCAGAAAACATGCC
CGTCGGCTACGGCGTTGACACCACCAGAAAAAATGTTGACTCCCTT
(ID SEQ N° 53).
 Cette séquence d'ADN, clonée dans pCR3.1, code donc pour un polypeptide IL1b sous contrôle du promoteur du CMV.

On réalise une première injection intra-musculaire en injectant 100 µg de vecteur dans une solution saline de 100 µl. On réalise 2 rappels à J21 et J40 avec le peptide de l'exemple 13 (immunisation du type "prime-boost"). A J60 la réponse la réponse anticorps est évaluée par test ELISA sur la cytokine native sur les 5 souris immunisées et les 5 souris contrôle.

Le résultat est le suivant :

| Souris | 1 | 2 | 3 | 4 | 5 | Moyenne |
|---|---|---|---|---|---|---|
| Peptide | 1.54 | 0.35 | 1.88 | 1.64 | 0.24 | 1.13 |
| Contrôle | 0.13 | 0.11 | 0.18 | 0.08 | 0.23 | 0.14 |

### Exemple 15.

Le vaccin est formé d'une émulsion eau dans huile constituée de 50 % d'ISA 51 (Seppic, Paris) et de 50 % d'une solution aqueuse de peptide de l'exemple 1 (50 µg/dose).

Exemple 16 : Vaccin à base de l'immunogène plasmidique pour vaccination ADN de type systémique IL10.

Des plasmides codant pour les peptides APVRSLNCTL et LHLQGQDMEQQ de l'IL1β (20 µg/dose) ont été mis en suspension dans 0,2 ml de PBS pour administration intramusculaire.

### Exemple 17.

On a préparé un vaccin formé d'une émulsion eau dans huile de 50 % d'ISA (SEPPIC, Paris) et de 50 % d'une solution aqueuse du peptide de synthèse KYQAEGQLQWLNRRANALLANGVELRDNQL dérivé de TNFα humain couplé au KLH (100 µg/dose).

## Revendications

1. Vaccin contenant un composé immunogène comprenant un peptide ou un dérivé dudit peptide par délétion, substitution, addition, cyclisation, modification stéréochimique (utilisation d'acides aminés de série D) ou fonctionnalisation (telle qu'acylation) d'un ou plusieurs acides aminés dudit peptide, dans lequel ledit peptide a une taille comprise entre 10 et 30 acides amines, et ayant 70% d'identité avec les sites épitopiques suivants provenant de cytokine :
1-MQDPYVKEAENLKKYFNAGHSDVADNGTLFLGILKN-36 (ID SEQ No. 8),
118-MAELSPAAKTGKRKRS-133 (ID SEQ No. 9),
20-PNMLRDLRDAFSRVKTFFQMKDQLDNLLLKE-50 (ID SEQ No. 10),
5-ITLQEIIKTLNSL-17 (ID SEQ No. 11),
135-KSSRGSSDPQG-145 (ID SEQ No. 14),
1-IPTSALVKETLALLSTHRTLLIANET-26 (ID SEQ No. 19),
57-CSAIEKTQRMLSGFCPHKVSAGQFSS-82 (ID SEQ No. 30),
115-LLPDSPVGQLHASLLGLSQ-133 (ID SEQ No. 32),
114-RAVQMSTKVLIQFLQKKAKNLDAITTPDPTTNASLL-149 (ID SEQ No. 26),
80-ISRIAVSYQTKVNLLS-95 (ID SEQ No. 6),
124-FQLEKGDRLSAEINR-138 (ID SEQ No. 7),
39-VEIIATMKKKGEKRCLNPESKA-60 (ID SEQ No. 18),
51-ADPSEEWVQKYVSDLELSA-69 (ID SEQ No. 27),
52-ADPSESWVQEYVYDLELN-69 (ID SEQ No. 28),
51-ANPEKKWVREYINSLEMS-68 (ID SEQ No. 34),
73-IMRIKPHQGQHIGEMS-88 (ID SEQ No. 4),
87-TILYYIGKTPKIEQ-100 (ID SEQ No. 22),
123-STSQAENMPV-132 (ID SEQ No. 3),
dans lequel ledit composé immunogène ne comporte pas d'autres épitopes de ladite cytokine et dans lequel ledit composé immunogène est capable de générer chez un sujet des anticorps reconnaissant la cytokine native.

2. Vaccin selon la revendication 1, dans lequel le peptide provient d'une cytokine Th1/Th2 choisie parmi IFN gamma, IL-10, IL-4, IL-12, IL-5, IL-13, IL-23, IL-6, et TNF alpha et le site épitopique est choisi parmi :
1-MQDPYVKEAENLKKYFNAGHSDVADNGTLFLGILKN-36 (ID SEQ No. 8),
118-MAELSPAAKTGKRKRS-133 (ID SEQ No. 9),
20-PNMLRDLRDAFSRVKTFFQMKDQLDNLLLKE-50 (ID SEQ No. 10),
5-ITLQEIIKTLNSL-17 (ID SEQ No. 11),
135-KSSRGSSDPQG-145 (ID SEQ No. 14),
1-IPTSALVKETLALLSTHRTLLIANET-26 (ID SEQ No. 19),
57-CSAIEKTQRMLSGFCPHKVSAGQFSS-82 (ID SEQ No. 30),
115-LLPDSPVGQLHASLLGLSQ-133 (ID SEQ No. 32),
114-RAVQMSTKVLIQFLQKKAKNLDAITTPDPTTNASLL-149 (ID SEQ No. 26).
80-ISRIAVSYQTKVNLLS-95 (ID SEQ No. 6),
124-FQLEKGDRLSAEINR-138 (ID SEQ No. 7).

3. Vaccin selon la revendication 1, dans lequel le peptide provient d'une cytokine proinflammatoire choisie parmi IL-6, IL-1 beta et TNF alpha et le site épitopique est choisi parmi :
114-RAVQMSTKVLIQFLQKKAKNLDAITTPDPTTNASLL-149 (ID SEQ No. 26).
123-STSQAENMPV-132 (ID SEQ No. 3),
80-ISRIAVSYQTKVNLLS-95 (ID SEQ No. 6),
124-FQLEKGDRLSAEINR-138 (ID SEQ No. 7),

4. Vaccin selon la revendication 1, dans lequel le peptide provient d'une cytokine choisie parmi VEGF et TGF beta et le site épitopique est choisi parmi :
73-IMRIKPHQGQHIGEMS-88 (ID SEQ No. 4),
87-TILYYIGKTPKIEQ-100 (ID SEQ No. 22).

5. Vaccin selon la revendication 1, dans lequel le peptide provient d'une chimiokine choisie parmi IP10, MIP 1 alpha, MIP 1 beta et Rantes et le site épitopique est choisi parmi :
39-VEIIATMKKKGEKRCLNPESKA-60 (ID SEQ No. 18),
51-ADPSEEWVQKYVSDLELSA-69 (ID SEQ No. 27),
52-ADPSESWVQEYVYDLELN-69 (ID SEQ No. 28),
51-ANPEKKWVREYINSLEMS-68 (ID SEQ No. 34).

6. Vaccin selon la revendication 1, dans lequel le peptide provient d'une cytokine choisie parmi IL-1 beta, IL6, et TNF alpha pour le traitement des maladies autoimmunes.

7. Vaccin selon la revendication 1, dans lequel le peptide provient d'une cytokine choisie parmi IFN gamma, IL-23 et IL-12 pour le traitement de la sclérose en plaques ou le diabète auto-immun, du psoriasis.

8. Vaccin selon la revendication 1, dans lequel le peptide provient d'une cytokine choisie parmi TNF alpha, IL-4, IL-5 et IL-13 pour le traitement de l'allergie ou de l'asthme.

9. Vaccin selon la revendication 1, dans lequel le peptide provient d'une cytokine choisie parmi IL-10, TGF beta, et VEGF pour le traitement du cancer.

10. Vaccin selon la revendication 1, dans lequel le peptide provient d'une cytokine choisie parmi IL-12, IL-2, IL-4, IL-6, IL-10, IL-13, IFN gamma et TNF alpha pour le traitement des dérèglements Th1/Th2.

11. Vaccin selon l'une quelconque des revendications précédentes dans lequel ledit peptide ou ledit dérivé est couplé à une protéine porteuse.

12. Vaccin selon la revendication 11 dans lequel ladite protéine porteuse est choisie parmi KLH, Tetanus Toxoid, transferrine et la ferritine.

13. Vaccin selon l'une quelconque des revendications précédentes comprenant en outre un adjuvant d'immunité minéral, huileux ou de synthèse.

14. Vaccin ADN codant pour un des peptides ou un des dérivés dudit peptide tel que défini à la revendication 1.

15. Anticorps contre un des peptides tel que défini à la revendication 1 pour une utilisation en immunothérapie passive.


SEQUENCE LISTING

<110>  Jean-François, ZAGURY

<120>  Peptides issus de cytokines et leur application en
thérapeutique

<130>  bif022175 pct

<160>  55

<170>  PatentIn version 3.2

<210>  1
<211>  10
<212>  PRT
<213>  Homo sapiens

<400>  1

Ala Pro Val Ala Ser Leu Ala Cys Thr Leu
1               5                   10

<210>  2
<211>  11
<212>  PRT
<213>  Homo sapiens

<400>  2

Leu His Leu Gln Gly Gln Asp Met Glu Gln Gln
1               5                   10

<210>  3
<211>  10
<212>  PRT
<213>  Homo sapiens

<400>  3

Ser Thr Ser Gln Ala Glu Asn Met Pro Val
1               5                   10

<210>  4
<211>  16
<212>  PRT
<213>  Homo sapiens

<400>  4

Ile Met Arg Ile Lys Pro His Gln Gly Gln His Ile Gly Glu Met Ser
1               5                   10                  15

<210>  5
<211>  35
<212>  PRT
<213>  Homo sapiens

<400>  5

Pro Gln Ala Glu Gly Gln Leu Gln Trp Leu Asn Arg Arg Ala Asn Ala
1               5                   10                  15

Leu Leu Ala Asn Gly Val Glu Leu Arg Asp Asn Gln Leu Val Val Pro
            20                  25                  30

Ser Glu Gly
        35

<210>  6
<211>  16
<212>  PRT
<213>  Homo sapiens

<400>  6

Ile Ser Arg Ile Ala Val Ser Tyr Gln Thr Lys Val Asn Leu Leu Ser
1               5                   10                  15

<210>  7
<211>  15
<212>  PRT
<213>  Homo sapiens

<400>  7

Phe Gln Leu Glu Lys Gly Asp Arg Leu Ser Ala Glu Ile Asn Arg
1               5                   10                  15

<210>  8
<211>  36
<212>  PRT
<213>  Homo sapiens

<400>  8

Met Gln Asp Pro Tyr Val Lys Glu Ala Glu Asn Leu Lys Lys Tyr Phe
1               5                   10                  15

Asn Ala Gly His Ser Asp Val Ala Asp Asn Gly Thr Leu Phe Leu Gly
            20                  25                  30

Ile Leu Lys Asn
        35

<210>  9
<211>  16
<212>  PRT
<213>  Homo sapiens

<400>  9

Met Ala Glu Leu Ser Pro Ala Ala Lys Thr Gly Lys Arg Lys Arg Ser
1               5                   10                  15

<210>  10
<211>  31
<212>  PRT
<213>  Homo sapiens

<400>  10

Pro Asn Met Leu Arg Asp Leu Arg Asp Ala Phe Ser Arg Val Lys Thr
1               5                   10                  15

Phe Phe Gln Met Lys Asp Gln Leu Asp Asn Leu Leu Leu Lys Glu
            20                  25                  30

<210>  11
<211>  13
<212>  PRT
<213>  Homo sapiens

<400>  11

Ile Thr Leu Gln Glu Ile Ile Lys Thr Leu Asn Ser Leu
1               5                   10

<210>  12
<211>  26
<212>  PRT
<213>  Homo sapiens

<400>  12

Ala Gln Gln Phe His Arg His Lys Gln Leu Ile Arg Phe Leu Lys Arg
1               5                   10                  15

Leu Asp Arg Asn Leu Trp Gly Leu Ala Gly
            20                  25

<210>  13
<211>  31
<212>  PRT
<213>  Homo sapiens

<400>  13

Leu Leu Leu His Lys Lys Glu Asp Gly Ile Trp Ser Thr Asp Ile Leu
1               5                   10                  15

Lys Asp Gln Lys Glu Pro Lys Asn Lys Thr Phe Leu Arg Cys Glu
            20                  25                  30

<210>  14
<211>  11
<212>  PRT
<213>  Homo sapiens

<400>  14

Lys Ser Ser Arg Gly Ser Ser Asp Pro Gln Gly
1               5                   10


<210>  15
<211>  35
<212>  PRT
<213>  Homo sapiens

<400>  15

Tyr Phe Gly Lys Leu Glu Ser Lys Leu Ser Val Ile Arg Asn Leu Asn
1               5                   10                  15


Asp Gln Val Leu Phe Ile Asp Gln Gly Asn Arg Pro Leu Phe Glu Asp
            20                  25                  30


Met Thr Asp
        35


<210>  16
<211>  11
<212>  PRT
<213>  Homo sapiens

<400>  16

Cys Glu Lys Ile Ser Thr Leu Ser Cys Glu Asn
1               5                   10


<210>  17
<211>  17
<212>  PRT
<213>  Homo sapiens

<400>  17

Glu Asp Glu Leu Gly Asp Arg Ser Ile Met Phe Thr Val Gln Asn Glu
1               5                   10                  15


Asp



<210>  18
<211>  24
<212>  PRT
<213>  Homo sapiens

<400>  18

Val Glu Ile Ile Ala Thr Met Lys Lys Lys Gly Glu Lys Arg Cys Arg
1               5                   10                  15


Cys Leu Asn Pro Glu Ser Lys Ala

            20

<210>  19
<211>  26
<212>  PRT
<213>  Homo sapiens

<400>  19

Ile Pro Thr Ser Ala Leu Val Lys Glu Thr Leu Ala Leu Leu Ser Thr
1               5                   10                  15

His Arg Thr Leu Leu Ile Ala Asn Glu Thr
            20                  25

<210>  20
<211>  13
<212>  PRT
<213>  Homo sapiens

<400>  20

Leu Gln Glu Phe Leu Gly Val Met Asn Thr Glu Trp Ile
1               5                   10

<210>  21
<211>  11
<212>  PRT
<213>  Homo sapiens

<400>  21

Lys Arg Asp Leu Gly Trp Lys Trp Ile His Glu
1               5                   10

<210>  22
<211>  14
<212>  PRT
<213>  Homo sapiens

<400>  22

Thr Ile Leu Tyr Tyr Ile Gly Lys Thr Pro Lys Ile Glu Gln
1               5                   10

<210>  23
<211>  13
<212>  PRT
<213>  Homo sapiens

<400>  23

Ala Asn Trp Val Asn Val Ile Ser Asp Leu Lys Lys Ile
1               5                   10

<210>  24

<211>  36
<212>  PRT
<213>  Homo sapiens

<400>  24

Ser Ser Asn Gly Asn Val Thr Glu Ser Gly Cys Lys Glu Cys Glu Glu
1               5                   10                  15

Leu Glu Lys Lys Asn Ile Lys Glu Phe Leu Gln Ser Phe Val His Ile
            20                  25                  30

Val Gln Met Phe
        35

<210>  25
<211>  12
<212>  PRT
<213>  Homo sapiens

<400>  25

Lys Gln Ile Arg Tyr Ile Leu Asp Gly Ile Ser Ala
1               5                   10

<210>  26
<211>  36
<212>  PRT
<213>  Homo sapiens

<400>  26

Arg Ala Val Gln Met Ser Thr Lys Val Leu Ile Gln Phe Leu Gln Lys
1               5                   10                  15

Lys Ala Lys Asn Leu Asp Ala Ile Thr Thr Pro Asp Pro Thr Thr Asn
            20                  25                  30

Ala Ser Leu Leu
        35

<210>  27
<211>  19
<212>  PRT
<213>  Homo sapiens

<400>  27

Ala Asp Pro Ser Glu Glu Trp Val Gln Lys Tyr Val Ser Asp Leu Glu
1               5                   10                  15

Leu Ser Ala

<210>  28
<211>  18
<212>  PRT
<213>  Homo sapiens

<400>  28

Ala Asp Pro Ser Glu Ser Trp Val Gln Glu Tyr Val Tyr Asp Leu Glu
1               5                   10                  15

Leu Asn

<210>  29
<211>  10
<212>  PRT
<213>  Homo sapiens

<400>  29

Thr Ala Leu Arg Glu Leu Ile Glu Glu Leu
1               5                   10

<210>  30
<211>  26
<212>  PRT
<213>  Homo sapiens

<400>  30

Cys Ser Ala Ile Glu Lys Thr Gln Arg Met Leu Ser Gly Phe Cys Pro
1               5                   10                  15

His Lys Val Ser Ala Gly Gln Phe Ser Ser
            20                  25

<210>  31
<211>  14
<212>  PRT
<213>  Homo sapiens

<400>  31

Gly His Met Asp Leu Arg Glu Glu Gly Asp Glu Glu Thr Thr
1               5                   10

<210>  32
<211>  19
<212>  PRT
<213>  Homo sapiens

<400>  32

Leu Leu Pro Asp Ser Pro Val Gly Gln Leu His Ala Ser Leu Leu Gly
1               5                   10                  15

Leu Ser Gln

<210> 33
<211> 20
<212> PRT
<213> Homo sapiens

<400> 33

Leu Leu Arg Phe Lys Ile Leu Arg Ser Leu Gln Ala Phe Val Ala Val
1               5                   10                  15

Ala Ala Arg Val
            20

<210> 34
<211> 18
<212> PRT
<213> Homo sapiens

<400> 34

Ala Asn Pro Glu Lys Lys Trp Val Arg Glu Tyr Ile Asn Ser Leu Glu
1               5                   10                  15

Met Ser

<210> 35
<211> 12
<212> PRT
<213> Homo sapiens

<400> 35

Arg Arg Thr Leu Met Leu Leu Ala Gln Met Arg Lys
1               5                   10

<210> 36
<211> 27
<212> PRT
<213> Homo sapiens

<400> 36

Leu Glu Ala Cys Val Ile Gln Gly Val Gly Val Thr Glu Thr Pro Leu
1               5                   10                  15

Met Lys Glu Asp Ser Ile Leu Ala Val Arg Lys
            20                  25

<210> 37
<211> 12
<212> PRT

<213>  Homo sapiens

<400>  37

Lys Leu His Leu Gln Gly Gln Asp Met Glu Gln Gln
1               5                   10


<210>  38
<211>  12
<212>  PRT
<213>  Homo sapiens

<400>  38

Lys Pro His Gln Gly Gln His Ile Gly Glu Met Ser
1               5                   10


<210>  39
<211>  30
<212>  PRT
<213>  Homo sapiens

<400>  39

Lys Tyr Gln Ala Glu Gly Gln Leu Gln Trp Leu Asn Arg Arg Ala Asn
1               5                   10                  15


Ala Leu Leu Ala Asn Gly Val Glu Leu Arg Asp Asn Gln Leu
            20                  25                  30


<210>  40
<211>  24
<212>  PRT
<213>  Homo sapiens

<400>  40

Lys Lys Tyr Phe Asn Ala Gly His Ser Asp Val Ala Asp Asn Gly Thr
1               5                   10                  15


Leu Phe Leu Gly Ile Leu Lys Asn
            20


<210>  41
<211>  29
<212>  PRT
<213>  Homo sapiens

<400>  41

Asp Glu Cys Asn Met Leu Arg Asp Leu Arg Asp Ala Phe Ser Arg Val
1               5                   10                  15


Lys Thr Phe Phe Gln Met Lys Asp Gln Leu Asp Asn Cys
            20                  25

<210>  42
<211>  19
<212>  PRT
<213>  Homo sapiens

<400>  42

Lys Gln Leu Ile Arg Phe Leu Lys Arg Leu Asp Arg Asn Leu Trp Gly
1               5                   10                  15

Leu Ala Gly

<210>  43
<211>  27
<212>  PRT
<213>  Homo sapiens

<400>  43

Lys Lys Glu Asp Gly Ile Trp Ser Thr Asp Ile Leu Lys Asp Gln Lys
1               5                   10                  15

Glu Pro Lys Asn Lys Thr Phe Leu Arg Cys Glu
            20                  25

<210>  44
<211>  24
<212>  PRT
<213>  Homo sapiens

<400>  44

Lys Tyr Phe Gly Lys Leu Glu Ser Lys Leu Ser Val Ile Arg Asn Leu
1               5                   10                  15

Asn Asp Gln Val Leu Phe Ile Asp
            20

<210>  45
<211>  17
<212>  PRT
<213>  Homo sapiens

<400>  45

Lys Lys Lys Gly Glu Lys Arg Lys Arg Cys Leu Asn Pro Glu Ser Lys
1               5                   10                  15

Ala

<210>  46
<211>  14

<212>  PRT
<213>  Homo sapiens

<400>  46

Lys Leu Gln Glu Phe Leu Gly Val Met Asn Thr Glu Trp Ile
1               5                   10


<210>  47
<211>  14
<212>  PRT
<213>  Homo sapiens

<400>  47

Asp Thr Ile Leu Tyr Tyr Ile Gly Lys Thr Pro Lys Ile Glu
1               5                   10


<210>  48
<211>  30
<212>  DNA
<213>  Homo sapiens

<400>  48
gcacctgtac gatcactgaa ctgcacgctc
30


<210>  49
<211>  32
<212>  DNA
<213>  Homo sapiens

<400>  49
ctccacctcc aggacaggat atggagcaac aa
32


<210>  50
<211>  30
<212>  DNA
<213>  Homo sapiens

<400>  50
agcacctctc aagcagaaaa catgcccgtc
30


<210>  51
<211>  45
<212>  DNA
<213>  Homo sapiens

<400>  51
cagtacatca aggctaactc caagttcatc ggtatcactg agctg
45


<210>  52
<211>  33
<212>  DNA

<213>  Homo sapiens

<400>  52
gttgacacca ccagaaaaaa tgttgactcc ctt
33


<210>  53
<211>  291
<212>  DNA
<213>  Homo sapiens

<400>  53
gttgacacca ccagaaaaaa tgttgactcc cttggctacg gcgcacctgt acgatcactg
60

aactgcacgc tcggctacgg cgttgacacc accagaaaaa atgttgactc ccttggctac
120

ggcctccacc tccagggaca ggatatggag caacaaggct acggccagta catcaaggct
180

aactccaagt tcatcggtat cactgagctg ggctacggca gcacctctca agcagaaaac
240

atgcccgtcg gctacggcgt tgacaccacc agaaaaaatg ttgactccct t
291


<210>  54
<211>  9
<212>  DNA
<213>  Homo sapiens

<400>  54
ggctacggc
9


<210>  55
<211>  16
<212>  PRT
<213>  Homo sapiens

<400>  55

Ala Gln Tyr Ile Lys Ala Asn Ser Lys Phe Ile Gly Ile Thr Glu Leu
1               5                   10                  15

SEQUENCE LISTING

<110>  VAXCONSULTING

<120>  Nouveaux peptides et leur application en thérapeutique

<130>  PCT/FR2003/001120

<140>  EP10183961.1
<141>  2010-09-30

<160>  55

<170>  PatentIn version 3.3

<210>  1
<211>  10
<212>  PRT
<213>  Artificial

<220>
<223>  peptide

<400>  1

Ala Pro Val Arg Ser Leu Asn Cys Thr Leu
1               5                   10


<210>  2
<211>  11
<212>  PRT
<213>  Artificial

<220>
<223>  peptide

<400>  2

Leu His Leu Gln Gly Gln Asp Met Glu Gln Gln
1               5                   10


<210>  3
<211>  10
<212>  PRT
<213>  Artificial

<220>
<223>  peptide

<400>  3

Ser Thr Ser Gln Ala Glu Asn Met Pro Val
1               5                   10


<210>  4
<211>  16
<212>  PRT
<213>  Artificial

<220>
<223>  peptide

<400>  4

Ile Met Arg Ile Lys Pro His Gln Gly Gln His Ile Gly Glu Met Ser
1               5                   10                  15

<210>  5
<211>  35
<212>  PRT
<213>  Artificial

<220>
<223>  peptide

<400>  5

Pro Gln Ala Glu Gly Gln Leu Gln Trp Leu Asn Arg Arg Ala Asn Ala
1               5                   10                  15

Leu Leu Ala Asn Gly Val Glu Leu Arg Asp Asn Gln Leu Val Val Pro
            20                  25                  30

Ser Glu Gly
        35

<210>  6
<211>  16
<212>  PRT
<213>  Artificial

<220>
<223>  peptide

<400>  6

Ile Ser Arg Ile Ala Val Ser Tyr Gln Thr Lys Val Asn Leu Leu Ser
1               5                   10                  15

<210>  7
<211>  15
<212>  PRT
<213>  Artificial

<220>
<223>  peptide

<400>  7

Phe Gln Leu Glu Lys Gly Asp Arg Leu Ser Ala Glu Ile Asn Arg
1               5                   10                  15

<210>  8
<211>  36
<212>  PRT
<213>  Artificial

<220>
<223>  peptide

<400>  8

Met Gln Asp Pro Tyr Val Lys Glu Ala Glu Asn Leu Lys Lys Tyr Phe
1               5                   10                  15

Asn Ala Gly His Ser Asp Val Ala Asp Asn Gly Thr Leu Phe Leu Gly
            20                  25                  30

Ile Leu Lys Asn
        35

<210>  9
<211>  16
<212>  PRT
<213>  Artificial

<220>
<223>  peptide

<400>  9

Met Ala Glu Leu Ser Pro Ala Ala Lys Thr Gly Lys Arg Lys Arg Ser
1               5                   10                  15

<210>  10
<211>  31
<212>  PRT
<213>  Artificial

<220>
<223>  peptide

<400>  10

Pro Asn Met Leu Arg Asp Leu Arg Asp Ala Phe Ser Arg Val Lys Thr
1               5                   10                  15

Phe Phe Gln Met Lys Asp Gln Leu Asp Asn Leu Leu Leu Lys Glu
            20                  25                  30

<210>  11
<211>  13
<212>  PRT
<213>  Artificial

<220>
<223>  peptide

<400>  11

Ile Thr Leu Gln Glu Ile Ile Lys Thr Leu Asn Ser Leu
1               5                   10

<210>  12
<211>  26
<212>  PRT
<213>  Artificial

<220>
<223>  peptide

<400>  12

Ala Gln Gln Phe His Arg His Lys Gln Leu Ile Arg Phe Leu Lys Arg
1               5                   10                  15

Leu Asp Arg Asn Leu Trp Gly Leu Ala Gly
            20                  25

<210>  13
<211>  31
<212>  PRT
<213>  Artificial

<220>
<223>  peptide

<400>  13

Leu Leu Leu His Lys Lys Glu Asp Gly Ile Trp Ser Thr Asp Ile Leu
1               5                   10                  15

Lys Asp Gln Lys Glu Pro Lys Asn Lys Thr Phe Leu Arg Cys Glu
            20                  25                  30

<210>  14
<211>  11
<212>  PRT
<213>  Artificial

<220>
<223>  peptide

<400>  14

Lys Ser Ser Arg Gly Ser Ser Asp Pro Gln Gly
1               5                   10

<210>  15
<211>  35
<212>  PRT
<213>  Artificial

<220>
<223>  peptide

<400>  15

Tyr Phe Gly Lys Leu Glu Ser Lys Leu Ser Val Ile Arg Asn Leu Asn
1               5                   10                  15

Asp Gln Val Leu Phe Ile Asp Gln Gly Asn Arg Pro Leu Phe Glu Asp
            20                  25                  30

Met Thr Asp
        35

<210>  16
<211>  11
<212>  PRT
<213>  Artificial

<220>
<223>  peptide

<400>  16

Cys Glu Lys Ile Ser Thr Leu Ser Cys Glu Asn
1               5                   10

<210>  17

<211>  17
<212>  PRT
<213>  Artificial

<220>
<223>  peptide

<400>  17

Glu Asp Glu Leu Gly Asp Arg Ser Ile Met Phe Thr Val Gln Asn Glu
1               5                   10                  15


Asp




<210>  18
<211>  22
<212>  PRT
<213>  Artificial

<220>
<223>  peptide

<400>  18

Val Glu Ile Ile Ala Thr Met Lys Lys Lys Gly Glu Lys Arg Cys Leu
1               5                   10                  15


Asn Pro Glu Ser Lys Ala
            20


<210>  19
<211>  26
<212>  PRT
<213>  Artificial

<220>
<223>  peptide

<400>  19

Ile Pro Thr Ser Ala Leu Val Lys Glu Thr Leu Ala Leu Leu Ser Thr
1               5                   10                  15


His Arg Thr Leu Leu Ile Ala Asn Glu Thr
            20                  25


<210>  20
<211>  13
<212>  PRT
<213>  Artificial

<220>
<223>  peptide

<400>  20

Leu Gln Glu Phe Leu Gly Val Met Asn Thr Glu Trp Ile
1               5                   10


<210>  21
<211>  11

<212>  PRT
<213>  Artificial

<220>
<223>  peptide

<400>  21

Lys Arg Asp Leu Gly Trp Lys Trp Ile His Glu
1               5                   10


<210>  22
<211>  14
<212>  PRT
<213>  Artificial

<220>
<223>  peptide

<400>  22

Thr Ile Leu Tyr Tyr Ile Gly Lys Thr Pro Lys Ile Glu Gln
1               5                   10


<210>  23
<211>  13
<212>  PRT
<213>  Artificial

<220>
<223>  peptide

<400>  23

Ala Asn Trp Val Asn Val Ile Ser Asp Leu Lys Lys Ile
1               5                   10


<210>  24
<211>  36
<212>  PRT
<213>  Artificial

<220>
<223>  peptide

<400>  24

Ser Ser Asn Gly Asn Val Thr Glu Ser Gly Cys Lys Glu Cys Glu Glu
1               5                   10                  15


Leu Glu Lys Lys Asn Ile Lys Glu Phe Leu Gln Ser Phe Val His Ile
            20                  25                  30


Val Gln Met Phe
        35


<210>  25
<211>  12
<212>  PRT
<213>  Artificial

<220>
<223>  peptide

<400>  25

Lys Gln Ile Arg Tyr Ile Leu Asp Gly Ile Ser Ala
1               5                   10


<210>  26
<211>  36
<212>  PRT
<213>  Artificial

<220>
<223>  peptide

<400>  26

Arg Ala Val Gln Met Ser Thr Lys Val Leu Ile Gln Phe Leu Gln Lys
1               5                   10                  15


Lys Ala Lys Asn Leu Asp Ala Ile Thr Thr Pro Asp Pro Thr Thr Asn
            20                  25                  30


Ala Ser Leu Leu
        35


<210>  27
<211>  19
<212>  PRT
<213>  Artificial

<220>
<223>  peptide

<400>  27

Ala Asp Pro Ser Glu Glu Trp Val Gln Lys Tyr Val Ser Asp Leu Glu
1               5                   10                  15


Leu Ser Ala



<210>  28
<211>  18
<212>  PRT
<213>  Artificial

<220>
<223>  peptide

<400>  28

Ala Asp Pro Ser Glu Ser Trp Val Gln Glu Tyr Val Tyr Asp Leu Glu
1               5                   10                  15


Leu Asn



<210>  29
<211>  10
<212>  PRT
<213>  Artificial

<220>
<223>  peptide

<400>  29

Thr Ala Leu Arg Glu Leu Ile Glu Glu Leu
1               5                   10


<210>  30
<211>  26
<212>  PRT
<213>  Artificial

<220>
<223>  peptide

<400>  30

Cys Ser Ala Ile Glu Lys Thr Gln Arg Met Leu Ser Gly Phe Cys Pro
1               5                   10                  15


His Lys Val Ser Ala Gly Gln Phe Ser Ser
            20                  25


<210>  31
<211>  14
<212>  PRT
<213>  Artificial

<220>
<223>  peptide

<400>  31

Gly His Met Asp Leu Arg Glu Glu Gly Asp Glu Glu Thr Thr
1               5                   10


<210>  32
<211>  19
<212>  PRT
<213>  Artificial

<220>
<223>  peptide

<400>  32

Leu Leu Pro Asp Ser Pro Val Gly Gln Leu His Ala Ser Leu Leu Gly
1               5                   10                  15


Leu Ser Gln



<210>  33
<211>  20
<212>  PRT
<213>  Artificial

<220>
<223>  peptide

<400>  33

Leu Leu Arg Phe Lys Ile Leu Arg Ser Leu Gln Ala Phe Val Ala Val
1               5                   10                  15

Ala Ala Arg Val
            20

<210>  34
<211>  18
<212>  PRT
<213>  Artificial

<220>
<223>  peptide

<400>  34

Ala Asn Pro Glu Lys Lys Trp Val Arg Glu Tyr Ile Asn Ser Leu Glu
1               5                   10                  15

Met Ser

<210>  35
<211>  12
<212>  PRT
<213>  Artificial

<220>
<223>  peptide

<400>  35

Arg Arg Thr Leu Met Leu Leu Ala Gln Met Arg Lys
1               5                   10

<210>  36
<211>  27
<212>  PRT
<213>  Artificial

<220>
<223>  peptide

<400>  36

Leu Glu Ala Cys Val Ile Gln Gly Val Gly Val Thr Glu Thr Pro Leu
1               5                   10                  15

Met Lys Glu Asp Ser Ile Leu Ala Val Arg Lys
            20                  25

<210>  37
<211>  12
<212>  PRT
<213>  Artificial

<220>
<223>  peptide

<400>  37

Lys Leu His Leu Gln Gly Gln Asp Met Glu Gln Gln
1               5                   10

<210>  38
<211>  12
<212>  PRT
<213>  Artificial

<220>
<223>  peptide

<400>  38

Lys Pro His Gln Gly Gln His Ile Gly Glu Met Ser
1               5                   10

<210>  39
<211>  30
<212>  PRT
<213>  Artificial

<220>
<223>  peptide

<400>  39

Lys Tyr Gln Ala Glu Gly Gln Leu Gln Trp Leu Asn Arg Arg Ala Asn
1               5                   10                  15

Ala Leu Leu Ala Asn Gly Val Glu Leu Arg Asp Asn Gln Leu
            20                  25                  30

<210>  40
<211>  24
<212>  PRT
<213>  Artificial

<220>
<223>  peptide

<400>  40

Lys Lys Tyr Phe Asn Ala Gly His Ser Asp Val Ala Asp Asn Gly Thr
1               5                   10                  15

Leu Phe Leu Gly Ile Leu Lys Asn
            20

<210>  41
<211>  29
<212>  PRT
<213>  Artificial

<220>
<223>  peptide

<400>  41

Asp Glu Cys Asn Met Leu Arg Asp Leu Arg Asp Ala Phe Ser Arg Val
1               5                   10                  15

Lys Thr Phe Phe Gln Met Lys Asp Gln Leu Asp Asn Cys

20                  25

<210>  42
<211>  19
<212>  PRT
<213>  Artificial

<220>
<223>  peptide

<400>  42

Lys Gln Leu Ile Arg Phe Leu Lys Arg Leu Asp Arg Asn Leu Trp Gly
1               5                   10                  15

Leu Ala Gly

<210>  43
<211>  27
<212>  PRT
<213>  Artificial

<220>
<223>  peptide

<400>  43

Lys Lys Glu Asp Gly Ile Trp Ser Thr Asp Ile Leu Lys Asp Gln Lys
1               5                   10                  15

Glu Pro Lys Asn Lys Thr Phe Leu Arg Cys Glu
            20                  25

<210>  44
<211>  24
<212>  PRT
<213>  Artificial

<220>
<223>  peptide

<400>  44

Lys Tyr Phe Gly Lys Leu Glu Ser Lys Leu Ser Val Ile Arg Asn Leu
1               5                   10                  15

Asn Asp Gln Val Leu Phe Ile Asp
            20

<210>  45
<211>  15
<212>  PRT
<213>  Artificial

<220>
<223>  peptide

<400>  45

Lys Lys Lys Gly Glu Lys Arg Cys Leu Asn Pro Glu Ser Lys Ala
1               5                   10                  15

<210>  46
<211>  14
<212>  PRT
<213>  Artificial

<220>
<223>  peptide

<400>  46

Lys Leu Gln Glu Phe Leu Gly Val Met Asn Thr Glu Trp Ile
1               5                   10


<210>  47
<211>  14
<212>  PRT
<213>  Artificial

<220>
<223>  peptide

<400>  47

Asp Thr Ile Leu Tyr Tyr Ile Gly Lys Thr Pro Lys Ile Glu
1               5                   10


<210>  48
<211>  30
<212>  DNA
<213>  Artificial

<220>
<223>  peptide encoding DNA

<400>  48
gcacctgtac gatcactgaa ctgcacgctc                                        30


<210>  49
<211>  33
<212>  DNA
<213>  Artificial

<220>
<223>  peptide encoding DNA

<400>  49
ctccacctcc agggacagga tatggagcaa caa                                    33


<210>  50
<211>  30
<212>  DNA
<213>  Artificial

<220>
<223>  peptide encoding DNA

<400>  50
agcacctctc aagcagaaaa catgcccgtc                                        30


<210>  51
<211>  45
<212>  DNA

<213>  Artificial

<220>
<223>  peptide encoding DNA

<400>  51
cagtacatca aggctaactc caagttcatc ggtatcactg agctg                    45


<210>  52
<211>  33
<212>  DNA
<213>  Artificial

<220>
<223>  peptide encoding DNA

<400>  52
gttgacacca ccagaaaaaa tgttgactcc ctt                                 33


<210>  53
<211>  291
<212>  DNA
<213>  Artificial

<220>
<223>  peptide encoding DNA

<400>  53
gttgacacca ccagaaaaaa tgttgactcc cttggctacg gcgcacctgt acgatcactg     60

aactgcacgc tcggctacgg cgttgacacc accagaaaaa atgttgactc ccttggctac    120

ggcctccacc tccagggaca ggatatggag caacaaggct acggccagta catcaaggct    180

aactccaagt tcatcggtat cactgagctg ggctacggca gcacctctca agcagaaaac    240

atgcccgtcg gctacggcgt tgacaccacc agaaaaaatg ttgactccct t             291


<210>  54
<211>  9
<212>  DNA
<213>  Artificial

<220>
<223>  peptide encoding DNA

<400>  54
ggctacggc                                                             9


<210>  55
<211>  16
<212>  PRT
<213>  Artificial

<220>
<223>  peptide

<400>  55

Ala Gln Tyr Ile Lys Ala Asn Ser Lys Phe Ile Gly Ile Thr Glu Leu
1               5                   10                  15